Europäisches Patentamt

European Patent Office

Office européen des brevets

(19)

(11) Veröffentlichungsnummer: **0 017 865**
**B1**

## (12) EUROPÄISCHE PATENTSCHRIFT

(45) Veröffentlichungstag der Patentschrift:
**25.11.81**

(21) Anmeldenummer: **80101786.4**

(22) Anmeldetag: **03.04.80**

(51) Int. Cl.³: **B 01 J 35/02**, B 01 J 23/22,
B 01 J 21/06, C 07 D 307/89 //
B01J35/04

(54) Vanadium- und titan- und/oder zirkonhaltiger Trägerkatalysator und seine Verwendung für die Herstellung von Phthalsäureanhydrid.

(30) Priorität: **11.04.79 DE 2914683**

(43) Veröffentlichungstag der Anmeldung:
**29.10.80 Patentblatt 80/22**

(45) Bekanntmachung des Hinweises auf die Patenterteilung:
**25.11.81 Patentblatt 81/47**

(84) Benannte Vertragsstaaten:
**AT BE DE FR GB IT**

(56) Entgegenhaltungen:
**DE-A-1 542 304**
**DE-B-2 510 994**
**FR-A-750 443**
**GB-A-893 987**
**US-A-2 408 164**

(73) Patentinhaber: **BASF Aktiengesellschaft,
Carl-Bosch-Strasse 38, D-6700 Ludwigshafen (DE)**

(72) Erfinder: **Reuter, Peter, Dr., Richard-Wagner-Strasse 7,
D-6702 Bad Duerkheim 1 (DE)**
Erfinder: **Blechschmitt, Kurt, Finkenstrasse 15,
D-6707 Schifferstadt (DE)**
Erfinder: **Wirth, Friedrich, Dr., Schlossgasse 6,
D-6700 Ludwigshafen (DE)**

Vanadium- und titan- und/oder zirkonhaltiger Trägerkatalysator und seine Verwendung
für die Herstellung von Phthalsäureanhydrid

Diese Erfindung betrifft einen neuen vanadium- und titan- und/oder zirkonhaltigen Trägerkatalysator und seine Verwendung für die Herstellung von Phthalsäureanhydrid durch katalytische Oxidation von o-Xylol oder Naphthalin.

Für die Herstellung von Phthalsäureanhydrid (nachfolgend »PSA« genannt) durch katalytische Oxidation von o-Xylol oder Naphthalin mit Luft oder einem sauerstoffhaltigen Gas sind schon vanadin- und titan- und/oder zirkonhaltige Trägerkatalysatoren vorgeschlagen worden, wie sie z. B. in der DE-C-1 442 590, DE-A-1 642 938 oder in der DE-B-2 510 994 beschrieben werden.

Man stellt sie durch Aufbringen eines und/oder Zirkondioxid und eine Vanadiumverbindung enthaltenden Gemisches auf einen Träger, der z. B. in Kugel-, Pillen-, Kegel- oder Ringform vorliegt, so her, daß der Träger nach Trocknung in einer Schichtdicke von etwa 0,02 bis 2 mm mit der aktiven Masse aus Vanadiumpentoxid und Titandioxid und/oder Zirkondioxid beschichtet ist.

Bewährt haben sich die kugelförmigen Katalysatoren und insbesondere die in der DE-B-2 510 996 beschriebenn ringförmigen Katalysatoren.

Für den Dauerbetrieb bei der PSA-Herstellung ist es wünschenswert, die Salzbadtemperatur zur Schonung des Katalysators möglichst niedrig zu halten. Aus wirtschaftlichen Gründen ist man auch an einer hohen Belastung des Katalysators interessiert.

Diesen Bemühungen sind z. B. dadurch Grenzen gesetzt, daß bei tieferen Temperaturen und höheren Belastungen der Phthalidgehalt im hergestellten PSA steigt. Da man die PSA-Qualität nach dem Phthalidgehalt bemißt, muß die Phthalidbildung so weitgehend wie möglich unterdrückt werden. Es war deshalb nach einem Katalysator zu suchen, der z. B. bei der Herstellung von PSA durch Oxidation von o-Xylol oder Naphthalin bei möglichst niedrigen Salzbadtemperaturen und erhöhter Belastung ein PSA von hoher Qualität liefert.

Es wurde nun gefunden, daß ein vanadium- und titan- und/oder zirkonhaltiger Trägerkatalysator, der durch Aufbringen eines Gemisches aus feinverteiltem Titandioxid und/oder Zirkondioxid und der Lösung oder Suspension einer Vanadinverbindung auf einen inerten, nichtporösen, ringförmigen Träger mit einem äußeren Durchmesser von 6 bis 10 mm, einer Länge von 4 bis 10 mm und einer Wandstärke von 0,5 bis 3 mm in der Weise erhalten wird, daß der Träger nach Trocknung in einer Schichtdicke von 0,05 bis 1 mm mit der aktiven Masse beschichtet ist, die 1 bis 30 Gewichtsprozent Vanadinpentoxid und 70 bis 99 Gew.-% Titandioxid und/oder Zirkondioxid enthält, die gewünschten Eigenschaften aufweist, dessen ringförmiger Träger an beiden Stirnflächen von innen nach außen so schräg abgekantet sind, daß die Länge der äußeren Zylinderwand gegenüber der Länge der inneren Zylinderwand um mindestens 20% verkürzt ist.

Dieser neue Katalysator ergibt z. B. bei der Herstellung von PSA aus o-Xylol oder Naphthalin hinsichtlich der Lebensdauer und der Belastbarkeit des Katalysators als auch bei der technischen Durchführung der Oxidationsreaktionen überraschende Vorteile.

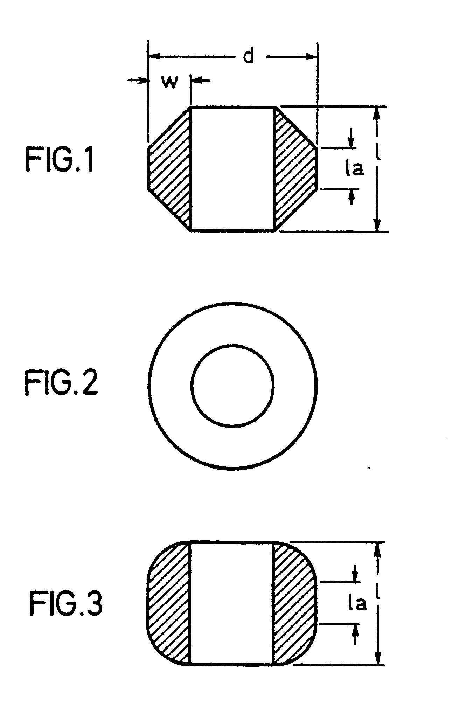

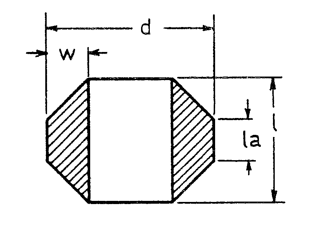

Die neuen Katalysatoren enthalten als Träger ringförmige Träger mit einem äußeren Durchmesser von 6 bis 10 mm, vorteilhaft von 7,3 bis 8,6 mm, eine Länge von 4 bis 10 mm, vorteilhaft von 5,3 bis 7,0 mm und eine Wandstärke von 0,5 bis 3 mm, vorteilhaft von 0,5 bis 2 mm. Wesentliches Merkmal der neuen Ringkatalysatoren ist, daß die beiden runden Stirnflächen des ringförmigen Trägers von innen nach außen so schräg abgekantet sind, daß die Länge der äußeren Zylinderwand gegenüber der Länge der inneren Zylinderwand um mindestens 20%, vorzugsweise um mindestens 50% verkürzt ist. Die Form der neuen Katalysatoren ist beispielsweise aus den Figuren ersichtlich, in denen der Katalysatorträger von der Seite (Fig. 1 und 3) und von oben (Fig. 2) dargestellt ist. Die schräg abgekanteten Stirnflächen können eben sein; vorteilhaft sind sie nach außen hin gewölbt. Durch die schräge Abkantung der beiden Stirnflächen ergibt sich die Verkürzung der Länge der äußeren Zylinderwand, die durch die Abkantung nicht betroffen ist. Die Länge dieser äußeren Zylinderwand (1a) beträgt z. B. 0 bis 8 mm, vorteilhaft 0,2 bis 5 mm.

Die Ringe bestehen aus inerten, nichtporösen Materialien, wie Eisen, Stahl, Aluminium, Porzellan, Tonerde, Aluminiumoxid oder Silikaten, wie Magnesium, Aluminium- oder Zirkonsilikat.

Die Beschichtung der Träger mit der katalytisch aktiven Masse nimmt man so vor, daß man auf den Träger ein Gemisch aus feinverteiltem Titandioxid und/oder Zirkondioxid und der Lösung oder Suspension einer Vanadinverbindung aufbringt. Als feinverteiltes Titandioxid verwendet man vorteilhaft Anatas mit einer spezifischen inneren Oberfläche von 5 bis 20 m²/g.

Als Vanadinverbindungen kommen Vanadinpentoxid oder solche Vanadinverbindungen in Betracht, die bei höherer Temperatur in Vanadinpentoxid übergehen, z. B. Vanadyloxalt, Vanadylformiat, Vanadyltartrat oder Ammoniumvanadat. Auch Mischfällungen von Titandioxid und Vanadinpentoxid oder Titan-Vanadium-Verbindungen, z. B. Titanvanadate, können eingesetzt werden. Das auf den Träger aufzubringende Gemisch wird auf an sich bekannte Weise z. B. so hergestellt, daß man das feinverteilte Titandioxid und/oder Zirkondioxid mit der Lösung oder Suspension der Vanadinverbindung in Wasser und/oder einem organischen Lösungsmittel, wie Formamid oder Äthanolamin mischt.

Das Beschichten selbst nimmt man vorteilhaft so vor, daß man das die katalytischen Substanzen enthaltende Gemisch auf den z. B. auf 110 bis 500°C erwärmten Träger aufbringt. Beim fertigen Katalysator macht die aktive Masse z. B. 4 bis 30 Gew.-% des Katalysators aus. Die Schichtdicke der aktiven Masse beträgt vorzugsweise 0,05 bis 0,5 mm.

Das die katalytisch aktiven Substanzen enthaltende Gemisch wird so eingestellt, daß die auf die Träger aufgebrachte aktive Masse nach Trocknung 1 bis 30, vorzugsweise 1 bis 15 Gew.-% Vanadinpentoxid und 70 bis 99 Gew.-%, vorzugsweise 85- bis 99 Gew.-% Titanoxid und/oder Zirkonoxid enthält. Die katalytisch aktive Masse kann z. B. bis zu einem Anteil von 5% auch noch andere Stoffe, wie die Oxide der Elemente Rubidium, Caesium, Phosphor oder Antimon, enthalten. In diesen Fällen werden dem auf die Träger aufzubringenden Gemisch entsprechende Mengen der genannten Oxide oder solcher Verbindungen zugegeben, die bei der Beschichtung in diese Oxide übergehen.

Die neuen Katalysatoren eignen sich z. B. für die Herstellung von Phthalsäureanhydrid durch Oxidation vn o-Xylol oder Naphthalin, für die Herstellung von Maleinsäureanhydrid durch Oxidation von Benzol oder ungesättigten aliphatischen $C_4$-Kohlenwasserstoffen, zur Herstellung von Pyromellithsäureanhydrid durch Oxidation aus Durol oder anderen 1,2,4,5-Tetraalkylbenzolen, zur Herstellung von Naphthalsäure aus Azenaphthen, zur Herstellung von Chinonen durch Oxidation von Naphthalin zu Naphthochinon oder von Anthracen, substituierten Indanen oder von Diphenylmethanverbindungen, wie z. B. von 2-Methyldiphenylmethan zu Anthrachinon mit Luft oder einem sauerstoffhaltigen Gas. Besonders vorteilhafte Ergebnisse werden bei der katalytischen Luftoxidation von o-Xylol oder Naphthalin zu PSA erhalten. Hierbei wird die Oxidation zweckmäßig auf an sich bekannte Weise in Röhrenreaktoren mit Salzbadkühlung bei Salzbadtemperaturen von 330 bis 450°C, vorzugsweise 340 bis 400°C, durchgeführt. Man kann die neuen Katalysatoren dabei mit 4 bis 10 Nm³ Luft bzw. sauerstoffhaltigem Trägergas je Stunde und Rohr (Durchmesser der Rohre etwa 16 bis 40 mm und Rohrlänge etwa von 1 bis 4 m) belasten. Dabei lassen sich Beladungen an o-Xylol oder Naphthalin von 20 bis 100 g/Nm³ anwenden.

Überraschenderweise wurde festgestellt, daß bei der Verwendung der erfindungsgemäßen Katalysatoren gegenüber den bekannten Katalysatoren bei der Oxidation von o-Xylol oder Naphthalin unter gleichen Versuchsbedingungen ein PSA von wesentlich besserer Qualität erhalten wird. Da das mit den erfindungsgemäßen Katalysatoren erhaltene PSA einen geringen Gehalt an Phthalid aufweist, ist es möglich, die neuen Katalysatoren bei tieferen Salzbadtemperaturen zu betreiben und länger bei tiefen Temperaturen zu halten. Temperaturerhöhungen, zu denen die allmähliche Alterung des Katalysators zwingt, können damit in größeren zeitlichen Abständen vorgenommen werden, als bei den bekannten Katalysatoren. Beides führt erfahrensgemäß zu längerer Lebensdauer des Katalysators und über die Lebensdauer des Katalysators zu einer insgesamt höheren Ausbeute.

Anstatt die Salzbadtemperatur zu senken, kann man bei den erfindungsgemäßen Katalysatoren auch die Belastung mit Luft oder dem Sauerstoff enthaltenden Trägergas im Vergleich zu den bekannten Katalysatoren erhöhen. Es ist auch möglich, die Luft oder das den Sauerstoff enthaltende Trägergas mit mehr o-Xylol/oder Naphthalin/Nm³ Luft oder Trägergas zu beladen als bei den bekannten Ringkatalysatoren, ohne daß sich die Qualität des erzeugten PSA verschlechtert. Dadurch wird eine energiesparendere Herstellung von PSA möglich.

Die größere Leistungsfähigkeit der neuen Katalysatoren zeigt ein Vergleich mit den bekannten Trägerkatalysatoren. Setzt man den erfindungsgemäßen Katalysator und den aus der DE-B-2 510 996 bekannten ringförmigen Katalysator bei der Oxidation von o-Xylol bei der gleichen Salzbadtemperatur ein, so erhält man ein PSA mit dem gleichen geringen Phthalidgehalt, wenn man bei einer Luftmenge von 4 Nm³/h · l Katalysator die o-Xylolbeladung bei dem bekannten Katalysator von 62 g/Nm³ Luft auf 85 g/Nm³ Luft beim erfindungsgemäßen Katalysator erhöht oder wenn man bei der gleichen Beladung von 62 g o-Xylol/Nm³ Luft die Luftmenge beim erfindungsgemäßen Katalysator auf 5,5 Nm³/h · l Katalysator steigert.

Beispiel 1

1000 g Steatit-Ringe mit einem äußeren Durchmesser (d) von 8 mm, einer Länge (l) von 6 mm und einer Wandstärke (w) von 1,5 mm, deren Stirnflächen ringsum nach außen hin so abgekantet sind, daß die Länge der äußeren Zylinderwand (la) 2,0 mm beträgt, werden in einer Dragiertrommel auf 260°C erhitzt. Bei dieser Temperatur wird eine Suspension, bestehend aus 400 g Anatas mit einer inneren Oberfläche von 11,2 m²/g; 73,6 g Vanadyloxalat (entspricht 30,1 g Vanadinpentoxid); 500 g Wasser und 100 g Formamid aufgesprüht, bis das Gewicht der aufgetragenen aktiven Masse 12,0% vom Gesamtgewicht des Katalysators ausmacht.

Die katalytische Masse besteht aus 7,0 Gew.-% Vanadinpentoxid und 93,0 Gew.-% Titandioxid. Die Schichtdicke beträgt im Durchschnitt 0,13 mm.

1150 g des so hergestellten Katalysators werden in ein senkrecht stehendes Eisenrohr von 25 mm lichter Weite und 3 m Länge gefüllt, das zur Temperaturregelung von einem Salzbad umgeben ist. Über diese Katalysatorschicht werden stündlich 230 g o-Xylol in Mischung mit 5500 l Luft bei 370°C geleitet.

Es wird PSA in einer Ausbeute von 115 Gew.-%, bezogen auf 100%iges o-Xylol, mit einem Phthalidgehalt von 0,012 Gew.-% erhalten.

### Vergleichsbeispiel

Verwendet man anstelle der in Beispiel 1 beschriebenen Ringe Steatit-Ringe mit einem äußeren Durchmesser von 8 mm, einer Länge von 6 mm, einer Wandstärke von 1,5 mm und mit nicht abgekanteten, sondern parallelen Stirnflächen, und verfährt man im übrigen wie im Beispiel beschrieben, so wird PSA in einer Ausbeute von 114,5 Gew.-%, bezogen auf 100%iges o-Xylol, mit einem Phthalidgehalt von 0,035 Gew.-% erhalten. Um den Phthalidgehalt im PSA auf 0,012 Gew.-% zu senken, muß die stündliche Belastung auf 184 g o-Xylol in Mischung mit 4500 l Luft zurückgenommen werden.

### Beispiel 2

#### a) Herstellung des Katalysators I

600 g Steatit-Ringe mit einem äußeren Durchmesser (d) von 8 mm, einer Länge (I) von 6 mm und einer Wandstärke (w) von 1,5 mm, deren Stirnflächen nach außen hin so abgekantet sind, daß die Länge der äußeren Zylinderwand (la) 2 mm beträgt, werden in einer Dragiertrommel auf 260°C erhitzt und mit einer Suspension, bestehend aus 400 g Anatas mit einer inneren Oberfläche von 11 m²/g, 73,2 g Vanadyloxalat (Vanadiumgehalt entspricht 41% $V_2O_5$), 500 g Wasser, 100 g Formamid und 1,09 g Rubidiumcarbonat besprüht, bis das Gewicht der aufgetragenen katalytischen Masse 10% vom Gesamtgewicht des Katalysators ausmacht. Die so aufgebrachte katalytisch aktive Masse besteht aus 0,202 Gew.-% Rubidiumoxid (entspricht 0,186 Gew.-% Rubidium), 7,0 Gew.-% Vanadinpentoxid und 92,84 Gew.-% Titandioxid, in der auf 35,3 Atome Vanadium 1 Atom Rubidium kommt. Der Rubidiumgehalt, bezogen auf Anatas, beträgt 0,20%.

#### b) Herstellung des Katalysators II

Man verfährt wie unter a) beschrieben, wobei man jedoch anstelle von Rubidiumcarbonat 4,87 g Ammoniumhydrogenphosphat zugibt. Im fertigen Katalysator macht das Gewicht der aufgetragenen Masse 10 Gew.-% vom Gesamtgewicht des Katalysators aus. Die katalytische Schicht besteht aus 0,3 Gew.-% Phosphor, 7,0 Gew.-% Vanadinpentoxid und 92,7 Gew.-% Titandioxid.

#### c) Verwendung der Katalysatoren

1,60 m des Katalysators II und anschließend 1,20 m des Katalysators I werden in ein 3,25 m langes Eisenrohr mit einer lichten Weite von 25 cm eingefüllt. Das Eisenrohr ist zur Temperaturregelung von einer Salzschmelze umgeben. Durch das Rohr werden stündlich von oben nach unten 382 g o-Xylol in Mischung mit 4500 l Luft bei 363°C geleitet.

Es wird PSA in einer Ausbeute von 114,1 Gew.-%, bezogen auf 100%iges o-Xylol, mit einem Phthalidgehalt von 0,015 Gew.-% erhalten.

### Vergleichsbeispiel

Verwendet man anstelle der im Beispiel 1 beschriebenen Ringe Steatit-Ringe mit einem äußeren Durchmesser von 8 mm, einer Länge von 6 mm, einer Wandstärke von 1,5 mm und mit nicht angekanteten, sondern parallelen Stirnflächen und verfährt man im übrigen wie im Beispiel 2 beschrieben, so wird PSA in einer Ausbeute von 114 Gew.-%, bezogen auf 100%iges o-Xylol, mit einem Phthalidgehalt von 0,081 Gew.-% erhalten. Um den Phthalidgehalt auf 0,015 Gew.-% zu senken, muß die stündliche Belastung auf 292 g o-Xylol in Mischung mit 4500 l Luft zurückgenommen werden.

### Beispiel 3

1000 g Steatit-Ringe mit einem äußeren Durchmesser (d) von 8 mm, einer Länge (I) von 6 mm und einer Wandstärke (w) von 1,5 mm, deren Stirnflächen nach außen hin so abgekantet sind, daß die Länge der äußeren Zylinderwand (la) 2 mm beträgt, werden in einer Dragiertrommel auf 260°C erhitzt und mit der in Beispiel 1 beschriebenen Suspension so lange besprüht, bis das Gewicht der aufgetragenen aktiven Masse 8,0% vom Gesamtgewicht des Katalysators ausmacht.

Die katalytische Masse besteht aus 7,0 Gew.-% Vanadinpentoxid und 93,0 Gew.-% Titandioxid. Die Schichtdicke beträgt im Durchschnitt 0,09 mm.

1130 g des so hergestellten Katalysators werden in ein senkrecht stehendes Eisenrohr von 25 mm lichter Weise und 3 m Länge gefüllt, das zur Temperaturregelung von einem Salzbad umgeben ist. Über diese Katalysatorschicht werden stündlich 160 g Naphthalin mit 4000 l Luft bei 372°C geleitet.

Es wird eine Ausbeute von 103,5 Gew.-%, bezogen auf 100%iges Naphthalin, erhalten. Der Naphthochinongehalt des erhaltenen PSA beträgt 0,3 Gew.-%.

### Vergleichsbeispiel

Verwendet man anstelle der in Beispiel 3 beschriebenen Katalysatorringe die entsprechenden Steatit-Ringe mit nicht abgekanteten Stirnflächen, so wird unter den in Beispiel 3 genannten Versuchsbedingungen ein PSA mit 0,8 Gew.-% Naphthochinon erhalten. Um den Naphthochinongehalt auf 0,3% zu reduzieren, muß die Salzbadtemperatur um 10°C auf 382°C angehoben werden.

### Patentansprüche

1. Vanadium- und titan- und/oder zirkonhaltiger Trägerkatalysator, erhalten durch Aufbringen eines Gemisches aus feinverteiltem Titandioxid und/oder Zirkondioxid und der Lösung oder Suspension einer Vanadinverbindung auf einen inerten, nicht porösen, ringförmigen Träger, mit einem äußeren Durchmesser von 6 bis 10 mm, einer Länge von 4 bis 10 mm und einer Wandstärke von 0,5 bis 3 mm, in der Weise, daß der Träger nach Trocknung in einer Schichtdicke von 0,05 bis 1 mm mit der aktiven Masse beschichtet ist, die 1 bis 30 Gew.-% Vanadinpentoxid und 70 bis 99 Gew.-% Titandioxid und/oder Zirkondioxid enthält, dadurch gekennzeichnet, daß die beiden Stirnflächen des ringförmigen Trägers von innen nach außen so schräg abgekantet sind, daß die Länge der äußeren Zylinderwand gegenüber der Länge der inneren Zylinderwand um mindestens 20% verkürzt ist.

2. Trägerkatalysator nach Anspruch 1, dadurch gekennzeichnet, daß die schräg abgekanteten Stirnflächen des ringförmigen Trägers nach außen hin gewölbt sind.

3. Verwendung des Trägerkatalysators nach Anspruch 1 für die Herstellung von Phthalsäureanhydrid durch katalytische Oxidation von o-Xylol oder Naphthalin.

### Claims

1. A supported catalyst containing vanadium and titanium and/or zirconium, obtained by applying a mixture of finely divided titanium dioxide and/or zirconium dioxide, and a solution or suspension of a vanadium compound, to an inert, non-porous, ring-shaped carrier having an external diameter of from 6 to 10 mm, a length of from 4 to 10 mm and a wall thickness of from 0.5 to 3 mm, in such a way that the carrier, after drying, carries a coating, from 0.05 to 1 mm thick, of the active material, containing from 1 to 30% by weight of vanadium pentoxide and from 70 to 99% by weight of titanium dioxide and/or zirconium dioxide, characterized in that the two end faces of the ring-shaped carrier are outwardly beveled at an angle such that the length of the outer cylindrical wall is at least 20% shorter than the length of the inner cylindrical wall.

2. A supported catalyst as claimed in claim 1, characterized in that the beveled end faces of the ring-shaped carrier are convex.

3. Use of a supported catalyst as claimed in claim 1 for the preparation of phthalic anhydride by catalytic oxidation of o-xylene or of naphthalene.

### Revendications

1. Catalyseur au vanadium et au titane et(ou) au zirconium sur support, obtenu par dépôt d'un mélange de bioxyde de titane et(ou) de bioxyde de zirconium finement dispersés et de la solution ou suspension d'un dérivé du vanadium sur un support inerte non poreux, de forme annulaire avec un diamètre extérieur de 6 à 10 mm, une longueur de 4 à 10 mm et une paroi d'une épaisseur de 0,5 à 3 mm, en des quantités telles que le support est muni, après séchage, d'une couche d'une épaisseur de 0,05 à 1 mm d'une masse active contenant 1 à 30% en poids de pentoxyde de vanadium et 70 à 99% en poids de bioxyde de titane et(ou) de bioxyde de zirconium, caractérisé en ce que les deux surfaces frontales du support annulaire sont biseautées de l'intérieur vers l'extérieur de telle façon que la longeuer de la paroi cylindrique extérieure est inférieure d'au moins 20% à la longueur de la paroi cylindrique intérieure.

2. Catalyseur sur support suivant la revendication 1, caractérisé en ce que les surfaces frontales biseautées du support annulaire sont concaves vers l'extérieur.

3. Utilisation d'un catalyseur sur support suivant la revendication 1 pour la préparation de l'anhydride phtalique par oxydation catalysée de l'ortho-xylène ou du naphtalène.

FIG.1

FIG.2

FIG.3